# EUROPEAN PATENT SPECIFICATION

(11) **EP 3 829 474 B1**
(45) Date of publication and mention of the grant of the patent: **28.06.2023**
(21) Application number: 19752431.7
(22) Date of filing: 26.07.2019
(51) Int. Cl.: A61B 18/18

(54) **ELECTROSURGICAL INSTRUMENT**
ELEKTROCHIRURGISCHES INSTRUMENT
INSTRUMENT ÉLECTRO-CHIRURGICAL

(30) Priority: 30.07.2018 GB 201812390
(43) Date of publication of application: 09.06.2021
(73) Proprietor: Creo Medical Limited, Chepstow, Wales NP16 5UH (GB)
(72) Inventor: HANCOCK, Christopher Paul, Bath, Bath and North East Somerset BA1 4LN (GB); PRESTON, Shaun, Chepstow Wales NP16 5UH (GB); BURN, Patrick, Chepstow Wales NP16 5UH (GB); TAPLIN, William, Chepstow Wales NP16 5UH (GB); JONES, Aeron W, Chepstow Wales NP16 5UH (GB); SWAIN, Sandra, Chepstow Wales NP16 5UH (GB); ULLRICH, George, Bangor Gwynedd LL57 4DB (GB); WEBB, David, Bangor Gwynedd LL57 4DB (GB)
(74) Representative: Mewburn Ellis LLP
(86) International application number: PCT/EP2019/070202
(87) International publication number: WO 2020/025481

(56) References cited:
- WO-A1-2017/103209
- US-A- 5 006 119
- US-A1- 2003 100 894
- US-A1- 2009 299 360
- US-A1- 2013 079 765
- US-A1- 2017 215 955

## Description

### FIELD OF THE INVENTION

The invention relates to a device for providing illumination and vision at the distal end of an electrosurgical instrument.

### BACKGROUND TO THE INVENTION

Conventional surgical scoping devices comprise an insertion tube that can be manoeuvred to a treatment site in a patient's body via a catheter or natural orifice. The insertion tube conveys components to the treatment site. In some examples, the insertion tube comprises an observation channel for conveying an illumination signal and returning an imaging signal, and a separate instrument channel for conveying an instrument for manipulating or otherwise treating tissue at the treatment site. It can be desirable to have real-time vision of the treatment site during treatment. In this context, reference is made to document US2013/0079765 A1.

Electrosurgical instruments are instruments that are used to deliver radiofrequency and/or microwave frequency energy to biological tissue, for purposes such as cutting biological tissue or coagulating blood. Radiofrequency and/or microwave frequency energy is typically supplied to the electrosurgical instrument using a cable. Conventional cables used for this purpose have a coaxial transmission line structure comprising a solid or multi-wire cylindrical inner conductor, a tubular layer of dielectric material around the inner conductor, and a tubular outer conductor around the dielectric material.

When operating many electrosurgical instruments it is common to need to provide additional supplies or components (e.g. control means) to the electrosurgical instrument, such as a liquid or gas feed, liquids or gases, or guide- or pullwires for manipulating (for example opening/closing, rotating or extending/ retracting) part(s) of the electrosurgical instrument.

In order to provide these additional supplies or components to the electrosurgical instrument, additional structures have been provided together with the conventional cable, such as additional tubes adjacent to the conventional cable. For example, it is known to provide an additional tube housing a pull-wire for the electrosurgical instrument alongside the conventional cable, and to house the conventional cable and the tube housing the pull-wire within a single protective jacket/casing.

Typically, the diameter of an instrument channel of a surgical scoping device (e.g. endoscope or laparoscope) is less than 3 mm, e.g. 2.8 mm. It is an ongoing challenge to provide both sufficient power and the additional supplies or components mentioned above in a compact enough form to fit within an instrument channel whilst maintaining flexibility and restricting power loss to acceptable (i.e. safe) levels.

### SUMMARY OF THE INVENTION

The invention and its preferred embodiments are defined in the appended set of claims. At its most general, the present invention proposes an improved system for obtaining images of a treatment site performing invasive electrosurgery. In one aspect, the invention provides a chip-based images sensor at the distal end of a cable that also conveys energy for electrosurgery. In another aspect, both light emitting and light sensing element are mounted at the distal end of a probe for insertion through the instrument channel of a surgical scoping device, whereby optical signals do not need to be conveyed through the scoping device, because the transition from electrical signals to optical radiation and back to electrical signals occurs at the distal end.

Advantages of the invention include the transmission of clear images enabling a clinician to better identify regions of tissue which require treatment, and enabling the clinician to more accurately perform treatment.

Particular embodiments of the invention propose combined delivery of RF and/or microwave frequency electromagnetic radiation for tissue treatment (e.g. ablation, coagulation or cutting) and transmission of digital images of the treatment site within a common structure that may form an imaging cable of a surgical scoping device. A common structure provides a more compact arrangement for systems in which it is desirable to visualise electrosurgical treatment.

In some embodiments, it can enable imaging or other forms of sensing to be available on surgical scoping devices without a dedicated observation channel. Furthermore, it can enable to the provision of ultra-small diameter surgical scoping devices opening up the possibility of electrosurgical treatment in regions that are inaccessible to conventional instruments.

The term "optical radiation" used herein may relate to electromagnetic radiation having a free space wavelength in the range 100 nm to 1 mm. In some embodiments, the optical radiation is in the visible spectrum, where it can be used to illuminate the treatment site and provide visual assistance for an operator. The optical radiation may be broadband, e.g. from a white light source. In other examples, the optical radiation may be narrow band or may have specific wavelengths for detecting or probing certain tissue characteristics.

According to the invention, there is provided an electrosurgical instrument for delivering radiofrequency (RF) and/or microwave energy into biological tissue, the electrosurgical instrument comprising: a coaxial cable for conveying radiofrequency (RF) and/or microwave energy, the coaxial cable having an inner conductor, an outer conductor formed coaxially with the inner conductor, and a first dielectric material separating the inner conductor and outer conductor, a radiating tip portion disposed at a distal end of the coaxial cable to receive the RF and/or microwave energy from the coaxial cable, and an image sensor disposed at the distal end of the coaxial cable for generating digital images of a treatment site.

In this way, the present invention allows for direct vision of a treatment site to enable a clinician to more accurately see and treat tissue within an operating region, while also providing RF and/or microwave energy for treatment with a radiating tip. The radiating tip portion may be delivered to the treatment site through a surgical scoping device such as a laparoscope, bronchoscope or the like.

Preferably, the image sensor is mounted at a distal end of an imaging cable. As explained in more detail below, it is envisaged that the coaxial cable may run through or alongside the imaging cable in some embodiments or, in other embodiments, the imaging cable may run through the coaxial cable. These arrangements in particular allow for a compact combined vision and electrosurgical treatment device. In particular, the combination device may be dimensioned to be insertable in a flexible insertion tube of an invasive surgical scoping device. For example, it may have a maximum outer diameter equal to or less than 3.5 mm, preferably equal to or less than 2.8 mm. Herein, the term "surgical scoping device" may be understood as a generic term that refers to a class of devices used in minimally invasive procedures, where the device typically include a rigid or flexible instrument cord that is insertable into a patient's body. The instrument cord is used to provide access to a treatment site for a variety of reasons, e.g. to perform surgical procedures, perform visual inspection or capture images, take biopsies, etc. Examples of a surgical scoping device include an endoscope, a bronchoscope, a laparoscope and the like.

The image sensor may be any suitable kind of chip-based image sensor, such as a digital camera. For example, the image sensor may be a charge-coupled device (CCD). Preferably the image sensor is a complementary metal-oxide-semiconductor (CMOS) sensor for capturing digital images of the treatment site. In particular, the CMOS may have 40 kilopixels, and may preferably have an area of 1 mm² or less. Such a sensor may provide high-quality images for a clinician while the small sensor area ensures that the device is insertable through a surgical scoping device.

Preferably, the imaging cable further comprises means for conveying optical radiation to illuminate the treatment site. The optical radiation may then be detected by the image sensor to provide high quality digital images of the treatment site.

For example, the imaging cable may comprise a bundle of optical fibres through which optical radiation may be transmitted from a proximal end of the electrosurgical instrument. Where optical fibres are used the electrosurgical instrument may also comprise a coupler at the proximal end of the imaging cable to couple optical radiation into the optical fibres for delivery to the distal end of the electrosurgical instrument at a treatment site.

In other examples, the electrosurgical instrument may comprise a light source mounted at the distal end of the imaging cable. For example, the light source may be a light emitting diode (LED). The LED may have a broad emission spectrum, for example it may be configured to emit white light for imaging, or the LED may be configured to emit in a narrow band. For example, the LED may have a narrow emission spectrum, or filters may be used to provide a narrow spectrum. In these arrangements, the imaging cable may advantageously be arranged to convey only electrical signals (e.g. power for the light source, and data from the image sensor). By conveying only electrical signals, the imaging cable need not convey optical radiation for the purpose of illumination or imaging. This may enable the imaging cable to be very compact, in particular as wires used to carry the electrical signal may have a smaller diameter than optical fibres used to convey optical radiation.

In some embodiments, the inner conductor of the coaxial cable may be hollow to form an optical channel, and the imaging cable may be located within the optical channel. The coaxial cable may comprise an innermost insulating layer between the inner conductive layer and the optical channel. Alternatively, an optical fibre bundle may form a ring, effectively providing an innermost insulating layer of the coaxial cable. The innermost insulating layer may prevent interference between the optical channel and the coaxial transmission line. Preferably, the coaxial cable may comprise a protective sheath on the outer surface of the outer conductor to protect the coaxial cable as it is inserted through a surgical scoping device. The protective sheath may be made from biocompatible material or may have a biocompatible coating. The protective sheath may contribute to steerability of the electrosurgical instrument. For example, the protective sheath may comprise a distal portion and a proximal portion, wherein the proximal portion is configured to have greater rigidity than the distal portion. The proximal portion may comprises an additional stiffening layer or braiding to inhibit flexing or deformation.

Preferably the optical channel extends through a bore in the radiating tip portion. In some examples the optical channel may terminate at an aperture formed in an outer surface of the radiating tip portion. This may ensure the clinician is provided with an accurate and useful view of the treatment site.

In other embodiments, the electrosurgical instrument may comprise an instrument cable for conveying the coaxial cable and the imaging cable, wherein the instrument cable comprises a working channel for conveying the coaxial cable. The optical channel may thus be nested within the working channel. Such an arrangement also provides advantages discussed above, in particular enabling the provision of ultra-small diameter electrosurgical instruments with combined vision and tissue treatment abilities. However, in other examples, the instrument cable may comprise a separate optical channel for conveying the imaging cable, wherein the optical channel runs adjacent to the working channel.

In some embodiments the light source and the image sensor may be detachably mounted at the distal end of the instrument cable. For example, the light source and the image sensor may be affixed to a removable structure which itself may be mounted within a distal end of the working channel and/or the optical channel. Optionally, the removable structure may form at least a distal portion of the working channel through which the coaxial cable may be fed.

Preferably, the instrument cable may comprise a protective sheath on its outer surface to protect the instrument cable as it is inserted through a surgical scoping device. The protective sheath may be made from biocompatible material or may have a biocompatible coating. The protective sheath may contribute to steerability of the electrosurgical instrument. For example, the protective sheath may comprise a distal portion and a proximal portion, wherein the proximal portion is configured to have greater rigidity than the distal portion. The proximal portion may comprises an additional stiffening layer or braiding to inhibit flexing or deformation.

Preferably, the coaxial cables comprises an innermost insulating layer, the inner conductor of the coaxial cable being formed on the innermost insulating layer. The coaxial cable may, in some embodiments, be incorporated into the wall of the working channel of the imaging cable and the innermost insulating layer may be hollow to form an instrument channel. The instrument channel may have a diameter of between 1 mm and 5 mm. The coaxial cable may alternatively be provided, at least in part, as a liner (e.g. detachable cover) for the working channel. The radiating instrument tip is provided at the distal end of the coaxial cable.

Preferably the coaxial cable may include a first terminal that is electrically connected to the inner conductor and which extends through the innermost insulating layer into the instrument channel; and a second terminal that is electrically connected to the outer conductor and which extends through the dielectric material and innermost insulating layer into the instrument channel. For example, the first terminal may be located proximally from the second terminal.

The radiating instrument tip may comprises means for connecting to the coaxial cable and the first and second terminals. For example, the radiating tip portion comprises: a first contact that is electrically connectable to the first terminal; a second contact that is electrically connected to the second terminal; and a distal bipolar transmission structure electrically connected to the first contact and the second contact for delivering the RF and/or microwave energy into biological tissue. Preferably wherein the distal bipolar transmission structure comprises a first conductive element that is electrically connected to the first contact and a second conductive element that is electrically connected to the first contact. Optionally, the first contact and the second contact are formed on a connection collar located proximally to the bipolar transmission structure.

The electrosurgical instrument may further comprise a handpiece which is provided for a clinician to operate the instrument. The coaxial cable and the imaging cable may be connected to the handpiece and extend away from it in a distal direction. For example, the handpiece may comprise a steering mechanism for controlling an orientation of a distal portion of the coaxial cable and/or the imaging cable, providing a clinician with a degree of control over the distal end of the instrument.

In some embodiments, the handpiece may comprise a light source and a coupler for coupling light emitted from the light source into a proximal end of the bundle of optical fibres.

The steering mechanism may comprise: an actuator mounted on an outer surface of the handpiece; a pull arm operably coupled to the actuator to slide within the handpiece and a control element extending along the coaxial cable and/or the imaging cable, the control element being operably coupled to the pull arm and the distal portion of the coaxial cable and/or the imaging cable. The control element may comprise one or more control wires that are attached to the pull arm and secured to the coaxial cable and/or the imaging cable at the distal portion thereof.

Preferably the handpiece may comprise a power source, for example a rechargeable power source. The power source may be used for powering the image sensor and/or the light source at the distal end of the electrosurgical device. Power from the power source may be transmitted to the image sensor and/or light source through a cable or wire. The cable or wire may run through the imaging cable. For example, the imaging cable may have an additional lumen for receiving the cable or wire which is separate from the working channel for receiving the coaxial cable.

Preferably the handpiece comprises a housing for enclosing its internal components. In some embodiments, the coaxial cable and/or imaging cable may be detachable from the housing.

Preferably, the handpiece comprises a communication module arranged to communicate information relating to the digital images to a remote device. For example, the communication module comprises a transceiver that is communicably connectable to a wireless network. Additionally or alternatively, the communication module may be arranged to upload image data to a remote server.

According to a further aspect of the invention, there is provided an electrosurgical apparatus. The apparatus comprises an electrosurgical instrument as described above, in addition to display device arranged to receive and display the information relating to detected optical radiation. In particular, the display device may be arranged to display the digital images captured by the image sensor. For example, the display device may be a laptop computer, tablet computer or a smartphone.

A further aspect of the invention provides an electrosurgical system comprising an electrosurgical generator arranged to generated RF and/or microwave EM energy, and an electrosurgical instrument as described above wherein the electrosurgical wherein the electrosurgical instrument is connected to the generator to receive the RF and/or microwave EM energy and couple it into the coaxial cable.

The use of optical radiation discussed herein need not be confined to providing images of the treatment site. The optical radiation can be used to probe the treatment site to measure properties thereof for diagnostic purposes. For example, the invention may be used to provide laser scattering measurements/spectroscopy, UV reflectometry/scattering measurements, etc.

In this specification "microwave" may be used broadly to indicate a frequency range of 400 MHz to 100 GHz, but preferably the range 1 GHz to 60 GHz. Specific frequencies that have been considered are: 915 MHz, 2.45 GHz, 3.3 GHz, 5.8 GHz, 10 GHz, 14.5 GHz and 24 GHz. The device may deliver energy at more than one of these microwave frequencies. In contrast, this specification uses "radiofrequency" or "RF" to indicate a frequency range that is at least three orders of magnitude lower, e.g. up to 300 MHz, preferably 10 KHz to 1 MHz.

References herein to a "conductor" or "conductive" material herein are to be interpreted as meaning electrically conductive unless the context makes clear that another meaning is intended.

### BRIEF DESCRIPTION OF THE DRAWINGS

Embodiments of the invention will now be described by way of example with reference to the accompanying drawings in which:
Fig. 1 is a schematic diagram of an electrosurgical apparatus that is an embodiment of the present invention;
Fig. 2 shows a front view of a first combined vision and treatment system;
Fig. 3 shows a cross section view of a coupler for coupling light into a bundle of optical fibres;
Fig. 4 shows a cross-sectional view of an instrument cable in one embodiment of the present invention;
Fig. 5 is a cross section view of an instrument tip for use with the present invention;
Fig. 6 is an exploded view of the instrument tip of Fig. 4;
Fig. 7 is a perspective view of a vision system that may be used in an aspect of the present invention;
Fig. 8 is a perspective view of a second combined treatment and vision system.

### DETAILED DESCRIPTION; FURTHER OPTIONS AND PREFERENCES

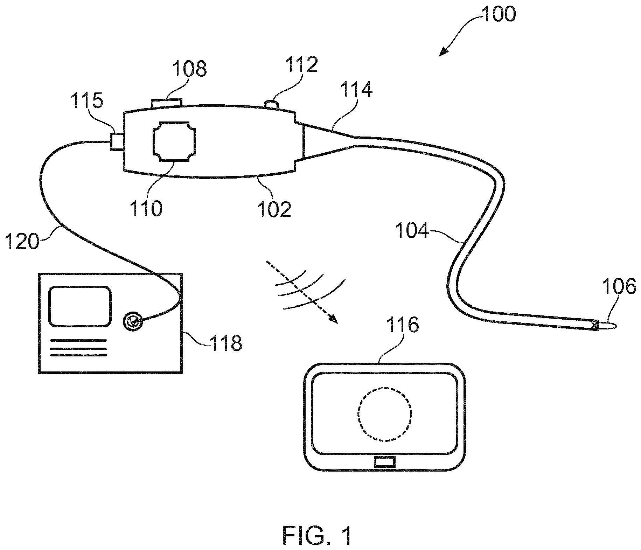

Fig. 1 is a schematic view of an electrosurgical apparatus 100 according to the present invention. The electrosurgical apparatus 100 comprises a handpiece 102 and a flexible instrument cable 104 extending away from the handpiece 102 in a distal direction. The flexible imaging cable is suitable for insertion into the body to access a treatment site. The flexible instrument cable 104 may have a biocompatible coating on its external surface so that it can be directly inserted into tissue. The instrument cable 104 may be introduced percutaneously or in a minimally invasive manner via a natural orifice. In some examples, the instrument cable 104 may be used with a separate surgical scoping device (not shown), such as a bronchoscope, endoscope, laparoscope or the like. In other examples, the imaging cable may be introduced through a guiding catheter. However, it may be particular advantageous for the imaging cable to be inserted directly (i.e. without surrounding components) to enable it to reach regions of the body that are difficult to access.

The instrument cable 104 in the invention has two functions: carrying microwave electromagnetic (EM) energy and/or radiofrequency (RF) EM energy to the treatment site, and carrying either optical radiation or energy for powering a light source for the purposes of imaging or sensing properties of the treatment site. As explained in more detail below, the instrument cable 104 of the invention provides these two functions in a particularly compact manner, by combining the two functions within a common structure. In some examples, an optical channel for conveying optical radiation or energy for powering a light source to and/or from the treatment site may be provided within an energy conveying means for the microwave and/or RF electromagnetic (EM) energy. For example, the optical channel may act as an observation channel arranged to carry optical signals to and from the treatment site to enable an image of the treatment site to be output from the handpiece 102. In other examples, an energy conveying structure for the microwave and/or RF EM energy may be provided within a working channel through an optical cable which is arranged to carry optical signals to and from the treatment site. These examples will be explained in more detail below.

The handpiece may include an observation port (not shown) for viewing the image. However, in a preferred arrangement, the handpiece 102 may be arranged to transmit the image to a separate display device 116. The image may be transmitted via a wireless connection, e.g. via WiFi or any other suitable networked communication configuration. The display device 116 may be any device with a display screen that is capable of receiving image data. The display device 116 may be portable, e.g. a laptop or tablet computer, a smartphone, or the like. The apparatus of the invention may include the display device, so that the benefits of the invention can be used in locations that do not have local display facilities.

An electrosurgical generator 118 is connected to the handpiece 102 via a cable 120 (e.g. a coaxial cable) which carries the RF and/or microwave energy into the handpiece 102. The generator 118 may be of the type described in WO 2012/076844, for example. The handpiece 102 comprising a connector port 115, which may be a QMA connector port or the like. The connector port 115 may be arranged to electrically connect the cable 120 to an energy conveying structure in the instrument cable 104. This electrical connection may be provided by a "T" connection between a coaxial cable from the generator and a coaxial transmission line of the energy conveying structure. Preferably there is a filter or choke between the "T" junction and an instrument port on the generator to prevent microwave leakage to the instrument port. This must be placed at half a wavelength at the microwave frequency from the "T" junction so that the "T" junction has a high return loss, i.e. does not reflect a significant proportion of the microwave energy back to the generator. The proximal end of the transmission line in the energy conveying structure is open circuit if RF energy is to be transmitted so as not to short out the RF voltage. It is also insulated and protected so that it does not break down for RF voltages or expose the operator to high RF voltages.

The instrument cable 104 has at its distal end a radiating tip 106 (or instrument tip) that is arranged to receive the RF and/or microwave energy from the energy conveying means in the insertion cable 104. The radiating tip 106 includes an energy delivery portion for delivering the received RF and/or microwave energy into biological tissue, e.g. to assist in treatment, e.g. cutting or coagulation.

The distal end of the instrument cable 104 may be steerable, e.g. to facilitate location of the radiating tip 106 in a desired position for treatment, and/or to enable optical radiation to be directed as desired, e.g. to obtain images of different parts of the treatment site or to take measurements in different positions. As explained below, in some examples the instrument cable 104 may include one of more control elements (e.g. e.g. pull/push rods or control wires) to facilitate steering. The control elements may pass out of a proximal end of the imaging cable to engage a steering mechanism mounted within the handpiece 102. The steering mechanism may be operable to extend and retract the control elements to effect action at the radiating tip. The steering mechanism may include an actuator mounted on the handpiece 102. In this example, the actuator is a rotatable knob 110. Rotation of the knob 110 relative to the housing can be converted to linear motion of the control element(s) via a suitable conversion mechanism mounted in the handpiece 102.

To limit the angle at which the proximal end of the instrument cable 104 can be bent relative to the handpiece 102, a conical restrictor 114 is fitted over the proximal end of the instrument cable 104. The conical restrictor 114 is secured to a distal end of the handpiece 102 and thus limits the movement of the cable to prevent it from experiences unwanted stresses.

The handpiece 102 comprises a housing that contains components associated with generating and controlling the optical radiation that can be conveyed along the optical channel in the instrument cable 104. For example, the handpiece 102 may contain a power source, such as a cell or other battery, an optical source, such as a light emitting diode (LED) or the like, and one or more optical elements for directing optical radiation from the optical source or from the treatment site in a desired manner. The optical elements may include a control interface 112 on the outer surface of the housing, to enable a user to control the optical elements in use. For example, the control interface 112 may control an intensity optical radiation delivered to the treatment site, or may control one or more lenses to assist in focussing an image signal received from the treatment site on to an optical sensor. In one example, an optical detector (e.g. a camera or the like) may be mounted in the handpiece to receive optical radiation returned from the treatment site in order to capture and transmit an image signal to the display device 116. In one example, the optical components may resemble a conventional fiberscope.

The handpiece 102 may include a power switch (not shown) for activating and deactivating the apparatus. The handpiece 102 may include a charging port (not shown) for connecting the power source to an external power supply to enable it to be recharged.

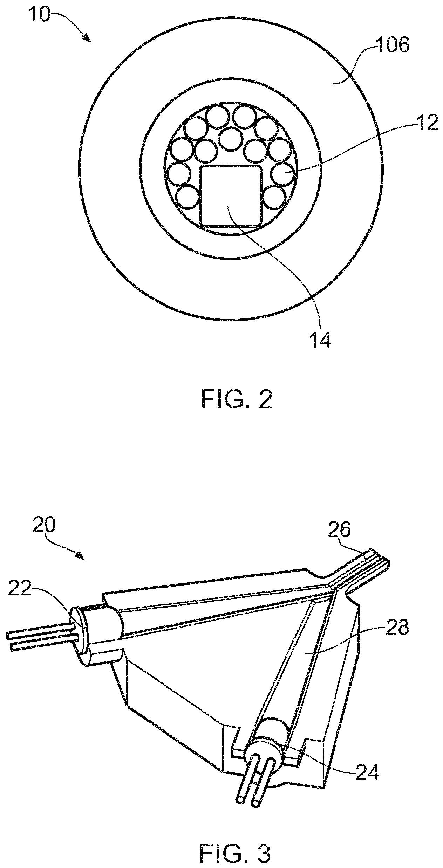

Fig. 2 shows a front view of a combination treatment and vision system 10 at the distal end of the instrument cable 104. The treatment and vision system 10 may be positioned in a central lumen of a radiating tip 106 which may be insertable through the instrument channel of a scoping device. The treatment and vision system 10 may provide illumination and vision at the distal end of the instrument cable 104 to aid a clinician.

The treatment and vision system 10 comprises an imaging cable, which in this example comprises a bundle of optical fibres 12 which are configured to provide illumination to the distal end of the instrument. The size, number and arrangement of the optical fibres 12 may be chosen to ensure optimal light levels. For example, the optical fibres 12 may be arranged in a complete ring around the image sensor 14, or may be arranged primarily on one side of the image sensor 14 as depicted in Fig. 2. In a preferred embodiment each optical fibre 12 has a diameter of 250 um, though the use of smaller optical fibres may allow more fibres to be placed into the central lumen of the radiating tip 200 and may therefore provide more light. Light may be coupled into a proximal end of the optical fibres 12 using collimating lenses or couplers, for example a coupler as shown in Fig. 3. It is envisaged that any suitable light source may be used, preferably using visible wavelengths. For example, specific wavelengths may be selected for narrow band imaging, such as a combination of 415 nm and 540 nm light, providing additional imaging modalities and allowing clinicians to see aspects of tissue in more detail and subsequently allow for easier identification of tissue lesions or other anomalies.

In some embodiments, a lens may be positioned over the bundle of optical fibres 12. The lens may be used to focus or diffuse light emitted from the optical fibres 12 depending on the application. Alternatively, the distal end of each of the optical fibres 12 may be shaped so as to direct light in a predetermined direction. For example, the distal end of an optical fibre 12 may form a ball lens, or may be tapered as required.

The image sensor 14 may be any chip-based image sensor suitable for detecting light at the selected frequencies. The image sensor 14 is configured to receive light at the distal end of the electrosurgical instrument and transmit images to the proximal end. For example, a cable may extend from the image sensor to the handpiece 102 where images are transmitted to a display 116, such as over Wi-Fi. Preferably the image sensor 14 is a CMOS sensor, though other suitable pixel sensors may be chosen to ensure that the display 116 receives a good image. In particular, the image sensor 14 may be a 40 kilopixel CMOS sensor having a surface area of 1 mm² or less. For example, a PICORAMEDIC (TM) sensor manufactured by Fujikura Ltd may be chosen as the image sensor 14.

The distal end of the combined treatment and vision system 10 is preferably deflectable to provide steerability and thus give a clinician greater control over the region which is imaged and ensure accurate treatment by the instrument tip 106. In one example, the system 10 may be provided through a steerable catheter, for example a catheter having a number of steering wires which give a clinician control over the distal end. Alternatively, the instrument cable 104 itself may comprise a number of steering wires to allow the distal end to be deflected. Deflection and steerability of the system 10 may be controlled by a clinician manipulating the handpiece.

Fig. 3 shows a cross-sectional view of a coupler 20 which may be positioned within the handpiece 102 of the electrosurgical apparatus 100 to provide light to a distal end of the instrument cable 104 through a bundle of optical fibres 12. The coupler 20 comprises two light source 22, 24 which may be LEDs. Each LED 22, 24 may have the same emission spectrum, or they may each be chosen to provide light at specific wavelengths. For example, LED 22 may provide light having a wavelength of 540 nm (green) and LED 24 may provide light having a wavelength of 425 nm (blue). This allows for narrow band imaging with at least two modalities allowing clinicians to see aspects of tissue in more detail and subsequently allow for easier identification of tissue lesions or other anomalies. Light from each light source 22, 24 is directed to output port 26 for coupling into the bundle of optical fibres 12 by a respected channel 28. The channels 28 may metallised to ensure good optical transmission of light from the light sources 22, 24. Circuitry within the handpiece 102 may provide for activation and dimming of each light source 22, 24 separately to tailor illumination to the response required by the clinician.

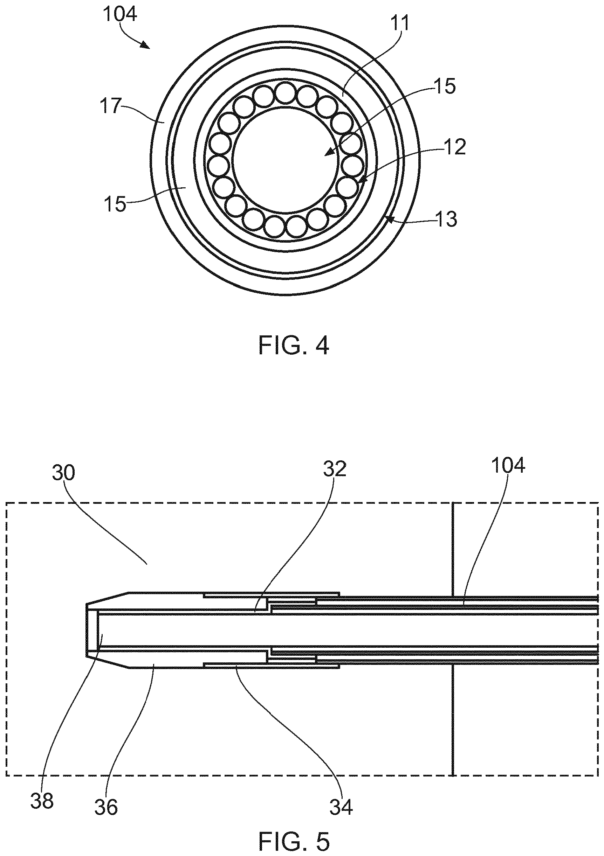

Fig. 4 shows a cross section through the instrument cable 104 in one embodiment of the invention. In this embodiment, the instrument cable 104 comprises a coaxial cable having an inner conductor 11, an outer conductor 13 formed coaxially with the inner conductor 11, and a first dielectric material 15 separating the inner conductor 11 and outer conductor 13. A protective sheath 17 is formed on the outside of the instrument cable 104 to protect the coaxial cable as it the instrument cable 104 is inserted through a surgical scoping device.

The inner conductor 11 is hollow to form an optical channel 15. The optical channel 15 carries an imaging cable formed from a bundle of optical fibres 12 and one or more wires (not shown) for supplying energy to an image sensor and for returning digital images to the handpiece of the electrosurgical instrument. As can be seen from Fig. 4, in this embodiment the bundle of optical fibres 12 form a ring on the inner surface of the inner conductor 11. In this way, the bundle of optical fibres 12 effectively form an innermost insulating layer of the coaxial cable, which may help reduce interference between digital image transmission through the optical channel and the RF and/or microwave energy conveyed through the instrument cable 104.

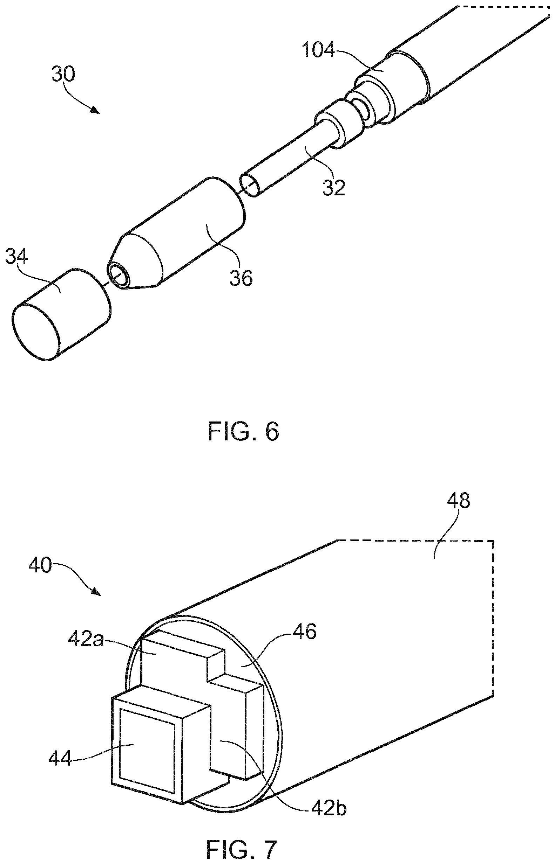

A cross sectional view of an radiating tip 30, which may be used as radiating tip 106 as described above, is shown in Fig. 5. An exploded view of the radiating tip 30 is shown in Fig. 6. The radiating tip 30 is configured to be mounted at the distal end of the instrument cable 104, wherein the instrument cable 104 comprises means for delivering RF and/or microwave frequency energy from its proximal end to the radiating tip 30. In particular, the instrument cable 104 may provide an energy conveying structure such as a coaxial cable, wherein the coaxial cable has an inner conductor, an outer conductor and a central lumen. The radiating tip 30 may thereby be used to perform electrosurgery, for example ablation.

The radiating tip 30 is a bipolar structure comprising an inner conductor 32, being a hollow metal cylinder connected to the inner conductor of the instrument cable 104. An outer conductor 34 is coaxial with the inner conductor 32 and is connected to the outer conductor of the coaxial cable within the instrument cable 104. The inner conductor 32 and outer conductor 34 of the instrument tip 30 are separated by a dielectric cylinder 36. The inner conductor 32 extends longitudinally through the dielectric cylinder beyond a distal end of the outer conductor 34. The radiating tip 30 provides a radiating antenna structure for RF and/or microwave frequency energy in order to treat tissue, e.g. by ablation. Each of the inner conductor 32 and outer conductor 34 preferably comprise silver or gold to ensure good microwave propagation.

The shape of the dielectric material 36 may also be selected to achieve efficient delivery of energy into tissue. For example, the dielectric 36 may be a cylindrical piece of ceramic material, polyether ether ketone (PEEK), or PTFE having a rounded distal tip.

The inner conductor 32 of the radiating tip 30 defines a central lumen 38 which collinear with the central lumen of the instrument cable 104. The central lumen 38 thus provides a channel through which an optical cable for providing illumination and vision may be fed, for example as discussed above with respect to Fig. 2 or below with respected to Fig. 6. For example, the central lumen 38 may have a diameter of 5 mm or less, for example 2 mm or less.

The radiating tip 30 may also have a biocompatible coating covering both the inner and outer surfaces of the radiating tip 30. The length of the radiating tip 30 is preferably selected to be a quarter wavelength at the predetermined operational frequency, preferably 5.8 GHz for example. The length should be minimised for insertion through scoping devices and to allow navigation through lumens within a patient's body.

Fig. 7 shows perspective view of a vision system 40 which may be provided at the distal end of an instrument cable 104. The vision system 40 may be used to provide combined vision and treatment, for example the vision system 40 may be positioned within a central lumen of an radiating tip (e.g. instrument tip 30 described above) in a similar may as described above with respect to the first vision system 10. It is also envisaged that the vision system 40 may be provided as a standalone illumination and vision system which may be delivered to a treatment site through an optical channel of an instrument cable, an imaging cable, or through a catheter such as a steerable catheter.

The vision system 40 comprises two light sources 42a, 42b which are provided in this embodiment as surface mount light emitting diodes (LEDs) each arranged generally along one side of an image sensor 44. Other arrangements of light sources 42a, 42b and image sensor 44 may be selected to provide optimal illumination of a treatment region. The image sensor 44 is preferably a CMOS sensor, such as a 40 kilopixel CMOS sensor having an area of less than 1 mm². The light sources 42a, 42b provide illumination for the image sensor 44 in place of optical fibres as described above. Each light source 42a, 42b may have the same emission spectrum, or may be chosen to each provide different wavelengths of light for multi-modal imaging of the treatment region.

A support structure 46 is provide to hold the LEDs 42a, 42b and image sensor 44 in their relative positions and strengthen the vision system 40 to allow the system 40 to be fed through a channel, such as the optical channel of an instrument cable. The support structure 46 may be made of any suitable biocompatible material which is robust enough to support the components of the vision system 30. For example, the support structure 40 may be made of a polymer material such as a polycarbonate. Openings within which the light sources 42a, 42b and the image sensor 44 are mounted may preferably be provided in the base material by laser cutting, though other manufacturing methods may also be suitable. The support structure 46 may have a diameter of less than 2 mm, such as 1.6 mm or less, to allow the vision system to be fed through an optical channel of an instrument cable 104, or an optical channel formed through a coaxial cable as described above.

The support structure 46 is provided at the distal end of a protective sheath 48 that conveys an imaging cable, which in this case comprises wires providing energy to the light sources 42a, 42b and image sensor 44 from the handpiece 102, and wires carrying the signal (e.g. image data) from the image sensor 44 to the handpiece 102 to be transmitted to a display. The protective sheath 48 is preferably made of a biocompatible material, and the distal end of the sheath 48 is potted to create a sealed enclosure to prevent the ingress of liquids or debris through or around the support structure 46.

The distal end of the vision system 40 is preferably deflectable to provide steerability and thus give a clinician greater control over the region which is imaged. In one example, the system 40 may be provided through a steerable catheter, for example a catheter having a number of steering wires which give a clinician control over the distal end. Alternatively, the instrument cable 104 itself may comprise a number of steering wires to allow the distal end to be deflected. Deflection and steerability of the system 10 may be controlled by a clinician manipulating the handpiece 102. In some embodiments, the protective sheath 48 may incorporate a number of steering wires which can be manipulated to deflect the vision system 40 by a clinician using the handpiece 102.

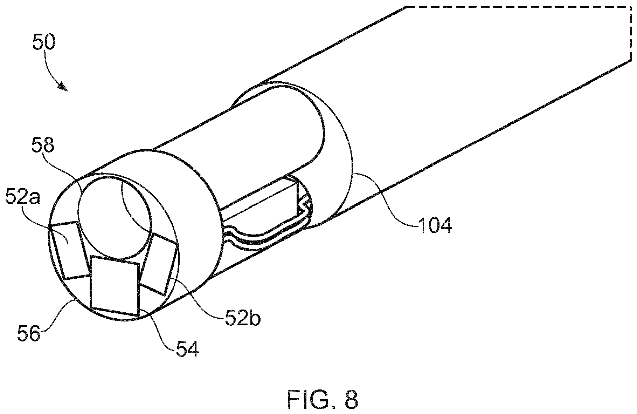

Fig. 8 shows a perspective view of a second combined treatment and vision system 50 according to an aspect of the present invention. The treatment and vision system 50 comprises two light sources 52a, 52b to provide illumination at the distal end of an imaging cable. For example, the light sources 52a, 52b may be light emitting diodes (LEDs) as described above with respect to Fig. 7. The light sources 52a, 52b are arranged symmetrically on either side of an image sensor 54, though any arrangement of the light sources 52a, 52b may be considered to provide optimal illumination of a treatment region. These components are held in place by a support structure 56.

The support structure 56 strengthens the distal end of the combined treatment and vision system 50 to ensure that the light sources 52a, 52b and image sensor 54 are held in their predetermined positions. The support structure 56 may be made of any suitable biocompatible material which is robust enough to support the components of the vision system 50. For example, the support structure 46 may be made of a polymer material such as a polycarbonate. Openings within which the light sources 52a, 52b and the image sensor 54 are mounted may preferably be provided in the base material by laser cutting, though other manufacturing methods may also be suitable. The support structure 56 also defines an opening 58 which is at the distal end of a working channel through the instrument cable 104. The working channel of the instrument cable 104 may be used to convey a coaxial cable, and/or to deliver tools and structures to a treatment site, and thus enables the system 50 to also provide treatment as required. The support structure 56 comprises a proximally extending extruded section connecting the working channel through the instrument cable 104 to the opening 58.

The support structure 56 may be detachably mounted to the distal end of the instrument cable 104. The support structure 56 may thus provide a removable structure allowing components to be easily changed by a clinician. For example, a clinician may wish to replace the support structure 56 to provide a different image sensor 54 and/or light sources 52a, 52b which are better suited to a particular treatment.

In some embodiments, the working channel may allow electrosurgery to be carried out at the distal end of the instrument cable 104. For example, a coaxial cable may be inserted through the working channel to provide RF and/or microwave frequency energy to an radiating tip at the opening 58 for electrosurgery. In other examples, the working channel may comprise an energy delivery structure which is incorporated into the sidewall of the working channel, and/or which is provided as a liner (such as a detachable cover) for the working channel. In such embodiments, the energy delivery structure may comprise a coaxial layered structure having an innermost insulating layer, an inner conductive layer formed on the innermost insulating layer, an outer conductive layer formed coaxially with the inner conductive layer, and a dielectric layer separating the inner conductive layer and the outer conductive layer. In this way, the energy delivery structure may provide a transmission line for conveying RF and/or microwave frequency energy to an instrument tip at the distal end of the working channel, at the opening 58. Preferably, the energy conveying structure may include, e.g. at a distal end thereof in a region proximate the opening 48, a first terminal that is electrically connected to the inner conductive layer and which extends through the innermost insulating layer into the working channel, and a second terminal that is electrically connected to the outer conductive layer and which extends through the dielectric layer and innermost insulating layer into the working channel. The first terminal and the second terminal may be arranged to form electrical connections (e.g. physically engage) with corresponding contacts formed on an electrosurgical instrument (e.g. a radiating tip) that is insertable in or through the working channel. The first terminal and the second terminal may be formed at the distal end of the inner conductive layer and outer conductive layer respectively, preferably in a region which is proximate the opening 48. The outer conductive layer may extend longitudinally further in a distal direction than the inner conductive layer, whereby the first terminal is located proximally from the second terminal.

The working channel of the instrument cable 104 for the vision system depicted in Fig. 7 may additionally or alternatively receive a catheter which provides for steerability at the distal end. For example, the catheter may comprise a number of steering wires extending from the handpiece 102 to the distal end of the working channel to enable a clinician to steer the instrument cable 104.

In addition to the working channel, the instrument cable 104 may comprise an optical channel for conveying wires and/or cables for delivery electricity from the handpiece 102 to power the light sources 52a, 52b and image sensor 54. The additional lumen also conveys wires and/or cables for conveying image signals from the image sensor 54 to the handpiece 102 to be transmitted to a display device 116.

The distal end of the combined treatment and vision system 50 is preferably deflectable to provide steerability and thus give a clinician greater control over the region which is imaged and ensure accurate treatment by an instrument tip. In one example, the system 50 may be provided through a steerable catheter, for example a catheter having a number of steering wires which give a clinician control over the distal end. Alternatively, the instrument cable 104 itself may comprise a number of steering wires to allow the distal end to be deflected. In another example, steering wires may be incorporated into the walls of the working channel, enabling deflection at the distal end of the instrument cable 104. Alternatively, the working channel may receive an articulated or knuckled guide wire in which sections of the guide wire may be adjusted to provide steerability of the combined treatment and vision system 50. Preferably, deflection and steerability of the system 50 may be controlled by a clinician manipulating the handpiece 102.

## Claims

1. An electrosurgical instrument for delivering radiofrequency (RF) and/or microwave energy into biological tissue, the electrosurgical instrument comprising:
a coaxial cable for conveying radiofrequency (RF) and/or microwave energy, the coaxial cable having an inner conductor (32), an outer conductor (34) formed coaxially with the inner conductor, and a first dielectric material separating the inner conductor and outer conductor,
a radiating tip portion (30) disposed at a distal end of the coaxial cable to receive the RF and/or microwave energy from the coaxial cable,
an image sensor (14, 44) disposed at the distal end of the coaxial cable for generating digital images of a treatment site, wherein the image sensor is mounted at a distal end of an imaging cable; and
a handpiece (102), wherein the coaxial cable and the imaging cable are connected to and extend away from the handpiece;
wherein the handpiece comprises a communication module arranged to communicate information relating to the digital images to a remote device, the communication module comprising a transceiver that is communicably connectable to a wireless network.

2. An electrosurgical instrument according to claim 1, wherein the image sensor is a complementary metal-oxide-semiconductor (CMOS) image sensor.

3. An electrosurgical instrument according to claim 1 or claim 2, wherein the imaging cable comprises a bundle of optical fibres for providing illumination at a treatment site.

4. An electrosurgical instrument according to claim 1 or claim 2, further comprising a light source mounted at the distal end of the imaging cable.

5. An electrosurgical instrument according to claim 4, wherein the light source comprises a light emitting diode.

6. An electrosurgical instrument according to any preceding claim, wherein the inner conductor of the coaxial cable is hollow to form an optical channel, and wherein the imaging cable is located within the optical channel.

7. An electrosurgical instrument according to claim 6, wherein the optical channel extends through a bore in the radiating tip portion.

8. An electrosurgical instrument according to claim 7, wherein the optical channel terminates at an aperture formed in an outer surface of the radiating tip portion.

9. An electrosurgical instrument according to any one of claims 1 to 5, comprising an instrument cable for conveying the coaxial cable and the imaging cable, wherein the instrument cable comprises a working channel for conveying the coaxial cable.

10. An electrosurgical instrument according to claim 9, wherein the instrument cable comprises an optical channel for conveying the imaging cable, wherein the optical channel runs adjacent to the working channel.

11. An electrosurgical instrument according to any preceding claim, wherein the coaxial cable and/or the imaging cable is detachable from the handpiece.

12. An electrosurgical instrument according to any preceding claim, wherein the communication module is arranged to upload image data to a remote server.

13. An electrosurgical apparatus comprising:
an electrosurgical instrument according to any preceding claim;
a display device arranged to receive and display the information relating to the digital images.

14. An electrosurgical system comprising:
an electrosurgical generator arranged to generate RF and/or microwave EM energy; and
an electrosurgical instrument according to any one of claims 1 to 12,
wherein the electrosurgical instrument is connected to the generator to receive the RF and/or microwave EM energy and couple it into the coaxial cable.

## Patentansprüche

1. Elektrochirurgisches Instrument zum Zuführen von Hochfrequenz- (HF-) und/oder Mikrowellenenergie in biologisches Gewebe, wobei das elektrochirurgische Instrument Folgendes umfasst:
ein Koaxialkabel zum Übertragen von Hochfrequenz- (HF-) und/oder Mikrowellenenergie, wobei das Koaxialkabel einen Innenleiter (32), einen Außenleiter (34), der koaxial mit dem Innenleiter ausgebildet ist, und ein erstes dielektrisches Material aufweist, das den Innenleiter und den Außenleiter trennt,
einen abstrahlenden Spitzenabschnitt (30), der an einem distalen Ende des Koaxialkabels angeordnet ist, um die HF- und/oder Mikrowellenenergie von dem Koaxialkabel zu erhalten,
einen Bildsensor (14, 44), der an dem distalen Ende des Koaxialkabels zum Erzeugen von digitalen Bildern einer Behandlungsstelle angeordnet ist,
wobei der Bildsensor an einem distalen Ende eines Bildgebungskabels montiert ist; und
ein Handstück (102),
wobei das Koaxialkabel und das Bildgebungskabel mit dem Handstück verbunden sind und sich davon weg erstrecken;
wobei das Handstück ein Kommunikationsmodul umfasst, das angeordnet ist, um Informationen in Bezug auf die digitalen Bilder an eine Fernvorrichtung zu kommunizieren, wobei das Kommunikationsmodul einen Sendeempfänger umfasst, der kommunizierbar mit einem Drahtlosnetzwerk verbindbar ist.

2. Elektrochirurgisches Instrument nach Anspruch 1, wobei der Bildsensor ein komplementärer Metalloxid-Halbleiter- (CMOS-) Bildsensor ist.

3. Elektrochirurgisches Instrument nach Anspruch 1 oder Anspruch 2, wobei das Bildgebungskabel ein Bündel optischer Fasern zum Bereitstellen einer Beleuchtung der Behandlungsstelle umfasst.

4. Elektrochirurgisches Instrument nach Anspruch 1 oder Anspruch 2, das ferner eine Lichtquelle umfasst, die an dem distalen Ende des Bildgebungskabels montiert ist.

5. Elektrochirurgisches Instrument nach Anspruch 4, wobei die Lichtquelle eine Leuchtdiode umfasst.

6. Elektrochirurgisches Instrument nach einem der vorangegangenen Ansprüche, wobei der Innenleiter des Koaxialkabels hohl ist, um einen optischen Kanal zu bilden, und wobei das Bildgebungskabel innerhalb des optischen Kanals angeordnet ist.

7. Elektrochirurgisches Instrument nach Anspruch 6, wobei sich der optische Kanal durch ein Loch in dem abstrahlenden Spitzenabschnitt erstreckt.

8. Elektrochirurgisches Instrument nach Anspruch 7, wobei der optische Kanal an einer Öffnung endet, die in einer Außenfläche des abstrahlenden Spitzenabschnitts ausgebildet ist.

9. Elektrochirurgisches Instrument nach einem der Ansprüche 1 bis 5, das ein Instrumentenkabel zum Führen des Koaxialkabels und des Bildgebungskabels umfasst, wobei das Instrumentenkabel einen Arbeitskanal zum Führen des Koaxialkabels umfasst.

10. Elektrochirurgisches Instrument nach Anspruch 9, wobei das Instrumentenkabel einen optischen Kanal zum Führen des Bildgebungskabels umfasst, wobei der optische Kanal benachbart zu dem Arbeitskanal verläuft.

11. Elektrochirurgisches Instrument nach einem der vorangegangenen Ansprüche, wobei das Koaxialkabel und/oder das Bildgebungskabel von dem Handstück abnehmbar sind.

12. Elektrochirurgisches Instrument nach einem der vorangegangenen Ansprüche, wobei das Kommunikationsmodul angeordnet ist, um Bilddaten an einen Fernserver hochzuladen.

13. Elektrochirurgische Vorrichtung, die Folgendes umfasst:
ein elektrochirurgisches Instrument nach einem der vorangegangenen Ansprüche;
eine Anzeigevorrichtung, die angeordnet ist, um die Informationen in Bezug auf die digitalen Bilder zu empfangen und anzuzeigen.

14. Elektrochirurgisches System, das Folgendes umfasst:
einen elektrochirurgischen Generator, der angeordnet ist, um HF- und/oder EM-Mikrowellen- Energie zu erzeugen; und
ein elektrochirurgisches Instrument nach einem der Ansprüche 1 bis 12,
wobei das elektrochirurgische Instrument mit dem Generator verbunden ist, um die HF- und/oder EM-Mikrowellen- Energie zu erhalten und diese in das Koaxialkabel zu koppeln.

## Revendications

1. Instrument électrochirurgical pour délivrer une énergie radiofréquence (RF) et/ou à micro-ondes dans un tissu biologique, l'instrument électrochirurgical comprenant :
un câble coaxial pour transporter de l'énergie radiofréquence (RF) et/ou à micro-ondes, le câble coaxial présentant un conducteur interne (32),
un conducteur externe (34) formé coaxialement avec le conducteur interne, et un premier matériau diélectrique séparant le conducteur interne et le conducteur externe,
une partie de pointe rayonnante (30) disposée à une extrémité distale du câble coaxial pour recevoir de l'énergie RF et/ou à micro-ondes à partir du câble coaxial,
un capteur d'image (14, 44) disposé à l'extrémité distale du câble coaxial pour générer des images numériques d'un site de traitement, dans lequel le capteur d'image est monté à une extrémité distale d'un câble d'imagerie ; et
une pièce à main (102), dans lequel le câble coaxial et le câble d'imagerie sont connectés à la pièce à main et s'étendent à l'écart de celle-ci ;
dans lequel la pièce à main comprend un module de communication agencé pour communiquer des informations relatives aux images numériques à un dispositif distant, le module de communication comprenant un émetteur-récepteur qui peut être connecté de manière communicante à un réseau sans fil.

2. Instrument électrochirurgical selon la revendication 1, dans lequel le capteur d'image est un capteur d'image à métal-oxyde-semiconducteur complémentaire (CMOS).

3. Instrument électrochirurgical selon la revendication 1 ou la revendication 2, dans lequel le câble d'imagerie comprend un faisceau de fibres optiques pour fournir un éclairage à un site de traitement.

4. Instrument électrochirurgical selon la revendication 1 ou la revendication 2, comprenant en outre une source de lumière montée à l'extrémité distale du câble d'imagerie.

5. Instrument électrochirurgical selon la revendication 4, dans lequel la source de lumière comprend une diode électroluminescente.

6. Instrument électrochirurgical selon l'une quelconque des revendications précédentes, dans lequel le conducteur interne du câble coaxial est creux pour former un canal optique, et dans lequel le câble d'imagerie est situé à l'intérieur du canal optique.

7. Instrument électrochirurgical selon la revendication 6, dans lequel le canal optique s'étend à travers un alésage dans la partie de pointe rayonnante.

8. Instrument électrochirurgical selon la revendication 7, dans lequel le canal optique se termine au niveau d'une ouverture formée dans une surface externe de la partie de pointe rayonnante.

9. Instrument électrochirurgical selon l'une quelconque des revendications 1 à 5, comprenant un câble d'instrument pour transporter le câble coaxial et le câble d'imagerie, dans lequel le câble d'instrument comprend un canal de travail pour transporter le câble coaxial.

10. Instrument électrochirurgical selon la revendication 9, dans lequel le câble d'instrument comprend un canal optique pour transporter le câble d'imagerie, dans lequel le canal optique s'étend de manière adjacente au canal de travail.

11. Instrument électrochirurgical selon l'une quelconque des revendications précédentes, dans lequel le câble coaxial et/ou le câble d'imagerie peuvent être détachés de la pièce à main.

12. Instrument électrochirurgical selon l'une quelconque des revendications précédentes, dans lequel le module de communication est agencé pour télécharger des données d'image vers un serveur distant.

13. Appareil électrochirurgical comprenant :
un instrument électrochirurgical selon l'une quelconque des revendications précédentes ;
un dispositif d'affichage agencé pour recevoir et afficher les informations relatives aux images numériques.

14. Système électrochirurgical comprenant :
un générateur électrochirurgical agencé pour générer de l'énergie RF et/ou à micro-ondes EM ; et
un instrument électrochirurgical selon l'une quelconque des revendications 1 à 12,
dans lequel l'instrument électrochirurgical est connecté au générateur pour recevoir l'énergie RF et/ou à micro-ondes EM et la coupler dans le câble coaxial.
